# EUROPÄISCHE PATENTSCHRIFT

(11) **EP 0 558 943 B1**
(45) Veröffentlichungstag und Bekanntmachung des Hinweises auf die Patenterteilung: **15.01.1997**
(21) Anmeldenummer: 93101685.1
(22) Anmeldetag: 04.02.1993
(51) Int. Cl.: C07C 29/10, C07C 35/48, C07C 35/52, C08G 63/682

(54) **Fluorsubstituierte vicinale Glykole, ein Verfahren zu ihrer Herstellung sowie ihre Verwendung**
Fluorosubstituted vicinal glycols, method for their preparation and their use
Glycols vinicaux fluorés, procédé pour leur préparation et leur utilisation

(30) Priorität: 29.02.1992 DE 4206384
(43) Veröffentlichungstag der Anmeldung: 08.09.1993
(73) Patentinhaber: HOECHST AKTIENGESELLSCHAFT, 65926 Frankfurt am Main (DE)
(72) Erfinder: Kulpe, Jürgen, Dr., W-6230 Frankfurt am Main 80 (DE); Strutz, Heinz, Dr., W-6230 Frankfurt am Main 80 (DE)

(56) Entgegenhaltungen:
- Keine einschl gigen Dokumente gefunden

## Beschreibung

Die vorliegende Erfindung betrifft fluorsubstituierte vicinale Glykole der allgemeinen Formel 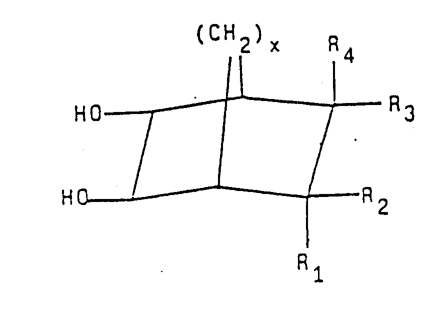
- in der: R₁ CF₃, F oder H,
R₂ CF₃, C₆F₁₃, C₈F₁₇, CH₂-C₆F₁₃, (CH₂)₂-C₆F₁₈
R₃ F oder H
R₄ F oder H
bedeuten
und x für 0 oder 1 steht,
sowie ein Verfahren zu ihrer Herstellung und ihre Verwendung.

Aus der großen Auswahl möglicher fluorsubstituierter vicinaler Glykole sind folgende Verbindungen von besonderem Interesse:
a) 2,3-Dihydroxy-5,5-bis(trifluormethyl)bicyclo[2.2.1]heptan, 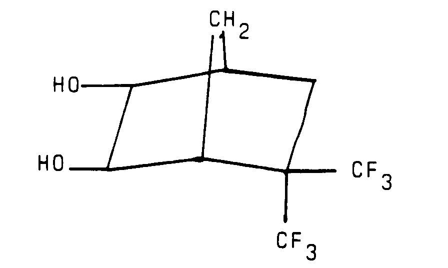
b) 2,3-Dihydroxy-5,6,6-trifluor-5-(trifluormethyl)bicyclo[2.2.1]heptan, 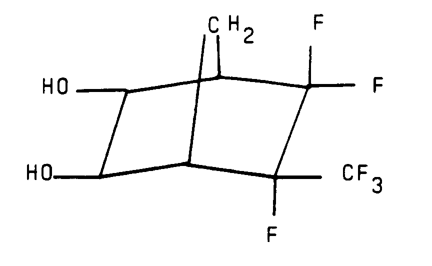
c) 2,3-Dihydroxy-5-(perfluorhexyl)bicyclo[2.2.1.]heptan, 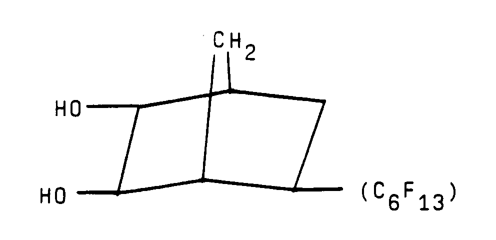
d) 2,3-Dihydroxy-5-(perfluoroctyl)bicyclo[2.2.1]heptan, 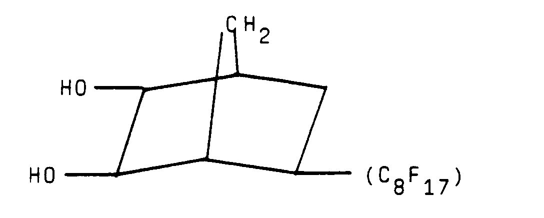
e) 1,2-Dihydroxy-4-(perfluorhexyl)cyclohexan, 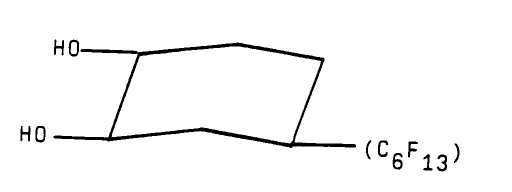
f) 2,3-Dihydroxy-5-(2,2,3,3,4,4,5,5,6,6,7,7,7-tridecafluorheptyl)bicyclo[2.2.1]heptan, 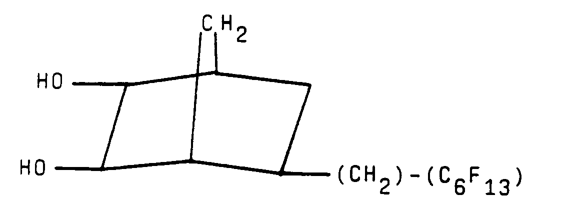
g) 2,3-Dihydroxy-5-(3,3,4,4,5,5,6,6,7,7,8,8,8-tridecafluoroctyl)bicyclo[2.2.1]heptan,

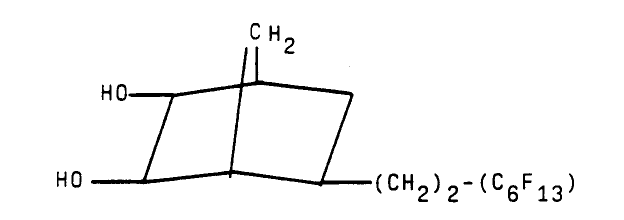

Als Ausgangsprodukte für die Herstellung fluorsubstituierter vicinaler Glykole wird auf das Umsetzungsprodukt von Butadien oder Cyclopentadien mit fluorsubstituiertem Alken zurückgegriffen. Die Umsetzung von Butadien bzw. Cyclopentadien mit Olefinen wird in der Literatur als "Diels-Alder-Reaktion" beschrieben. Im J. Am. Chem. Soc. (1955) Vol. 77, Pag. 915-919 und J. Org. Chem. (1973) Vol. 38, No. 11, Pag. 2o27-2o42 sind Reaktionen zur Herstellung von Bicyclo[2.2.1]hepten-Derivaten beschrieben, welche Fluor enthalten und als Einsatzstoffe für die Herstellung der fluorsubstituierten vicinalen Glykole geeignet sind.

Das Verfahren zur Herstellung der erfindungsgemäßen fluorsubstituierten vicinalen Glykole aus dem Umsetzungsprodukt von Butadien oder Cyclopentadien mit fluorsubstituiertem Alken nach der Arbeitsweise einer Diels-Alder-Reaktion ist dadurch gekennzeichnet, daß man das Umsetzungsprodukt in Gegenwart einer Säure mit Wasserstoffperoxid bei Temperaturen zwischen 4o und 1oo °C zur Reaktion bringt und das fluorsubstituierte vicinale Glykol isoliert.

Das Verfahren zur Herstellung der erfindungsgemäßen fluorsubstituierten vicinalen Glykole kann wahlweise und bevorzugt dadurch gekennzeichnet sein, daß man
1) 3o bis 7o %iges Wasserstoffperoxid in einer Menge von 1 bis 5 Äquivalenten Oxidationsmittel, bezogen auf das eingesetzte Diels-Alder-Umsetzungsprodukt, einsetzt,
2) als Säure Ameisen-, Essig-, Propionsäure oder wäßrige Schwefelsäure zusammen mit einer katalytischen Menge von [CH₃N(C₈H₁₇)₃][PO₄{W(O)(O₂)₂} ₄] einsetzt,
3) das Diels-Alder-Umsetzungsprodukt mit der 0,1- bis 1o-fachen Säuremenge vermischt,
4) die Reaktion in 1 bis 5o gewichtsprozentiger Schwefelsäure durchführt,
5) 0,1 bis 1o Molprozente [CH₃N(C₈H₁₇)₃][PO₄{W(O)(O₂)₂}₄], bezogen auf das Diels-Alder-Umsetzungsprodukt, einsetzt,
6) das fluorsubstituierte vicinale Glykol durch eine Sublimation reinigt,
7) das säurehaltige fluorsubstituierte vicinale Glykol mit Natronlauge versetzt, die wäßrige Phase mit Methylenchlorid extrahiert, den Extrakt mit der organischen Phase vereinigt, trocknet und danach das Methylenchlorid abdestilliert.

Die erfindungsgemäßen fluorsubstituierten vicinalen Glykole können zur Herstellung von Polyestern verwendet werden, in dem man fluorsubstituierte vicinale Glykole mit Dicarbonsäuren kondensiert. Diese Produkte zeichnen sich durch eine höhere thermische Beständigkeit aus.
Eine höhere thermische Beständigkeit von Polyestern wird auch schon erreicht, wenn man 5 bis 5o Molprozente fluorsubstituierte vicinale Glykole in Mischung mit Glykolen zusammen mit Dicarbonsäuren kondensiert.

Bei der gewählten Herstellungsart des Diels-Alder-Umsetzungsproduktes entsteht ein Isomerengemisch aus exo- und endo-Additionsprodukten. Es können aber auch die reinen exo- oder endo-Additionsprodukte einer Diels-Alder-Reaktion als Ausgangsprodukt für die Herstellung fluorsubstituierter vicinaler Glykole eingesetzt werden.

### Beispiel A (Diels-Alder-Reaktion)

In einem 1 l Autoklav werden 554 ml = 864 g (2,5 mol) Perfluorhexylethen, 165 g (2,5 mol) frisch destilliertes Cyclopentadien und 5 g Hydrochinon vorgelegt. Der Autoklav wird zweimal mit Stickstoff gespült. Anschließend werden 5 bar Stickstoff aufgedrückt und der Autoklav auf 17o °C erwärmt.

Nach 72 Stunden läßt man abkühlen, entspannt den Stickstoffüberdruck und filtriert den Autoklaveninhalt in eine Destillationsvorlage. Bei der fraktionierenden Vakuumdestillation werden 783 g des Diels-Alder-Produktes mit einem Siedepunkt von 33 - 35 °C bei einem Druck von 0,25 Torr isoliert. Durch ¹H-NMR-Spektroskopie wurde ein Endo/Exo-Isomerenverhältnis von 77/23 bestimmt.

Die nachfolgenden Beispiele sollen die Herstellung der erfindungsgemäßen Verbindungen erläutern.

### Beispiel 1

### Herstellung von 2,3-Dihydroxy-5,5-bis(trifluormethyl)bicyclo[2.2.1]heptan

5,2 g (2o mmol) des Diels-Alder-Umsetzungsproduktes von 3,3,3-Trifluor-2-trifluormethyl-1-propen (Hexafluorisobuten) und Cyclopentadien werden mit 2o ml Ameisensäure gemischt und bei 5o °C 1,3 ml 7o prozentiges Wasserstoffperoxid (3o mmol) zugegeben und 2o Stunden bei 5o °C gehalten. Ein Test auf Oxidationsmittel mit KI-Stärkepapier zeigte den vollständigen Abbau des Oxidationsmittels an. Die Leichtsieder werden im Vakuum abdestilliert. Der Rückstand wird mit 2oo ml 2o %iger Natronlauge auf 5o °C erwärmt. Der hierbei abgeschiedene Feststoff wird mit insgesamt 13o ml Methylenchlorid extrahiert. Die vereinigten Extrakte werden mit Magnesiumsulfat getrocknet, das Methylenchlorid abdestilliert und der Rückstand bei 6o °C im Ölpumpenvakuum sublimiert.
Ausbeute: 3,28 g = 62 % der Theorie.
Schmelzpunkt: 88 - 1oo °C.

### Beispiel 2

### Herstellung von 2,3-Dihydroxy-5,6,6-trifluor-5-(trifluormethyl)-bicyclo[2.2.1]heptan

4,3 g (2o mmol) des Diels-Alder-Umsetzungsproduktes von Hexafluorpropen und Cyclopentadien werden mit 1o ml Wasser, 1 ml 3o prozentiger Schwefelsäure und 4oo mg des Katalysators [CH₃N(C₈H₁₇)₃][PO₄ {W(O)(O₂)₂} ₄], dessen Herstellung in Synthesis 296 (1989) beschrieben ist, gemischt und auf 6o °C erwärmt. Dann werden 1,7 ml (4o mmol) 7o prozentiges Wasserstoffperoxid zugegeben und die Emulsion 6 Stunden lang bei 6o °C gehalten. Beim Abkühlen scheidet sich ein weißer Feststoff ab, der abgesaugt und mit kaltem Wasser gewaschen wird. Anschließend wird das Rohprodukt bei einem Druck von 0,1 mbar und einer Temperatur von 5o °C durch Sublimation gereinigt.
Ausbeute: 4,75 g = 94 % der Theorie.

### Beispiel 3 a

### Herstellung von 2,3-Dihydroxy-5-(perfluorhexyl)bicyclo[2.2.1]-heptan

8,2 g (2o mmol) des Diels-Alder-Umsetzungsproduktes von 3,3,4,4,5,5,6,6,7,7,8,8,8-Tridecafluor-1-octen (Perfluor-hexylethen) und Cyclopentadien werden mit 2o ml Ameisensäure gemischt und bei 6o °C 1,3 ml 7o prozentiges Wasserstofperoxid (3o mmol) zugesetzt und der Ansatz 36 Stunden lang bei 6o °C gehalten. Im Vakuum werden die Leichtsieder abdestilliert. Der Rückstand wird in 3 Portionen mit insgesamt 13o ml Methylenchlorid extrahiert. Die vereinigten Extrakte werden mit Magnesiumsulfat getrocknet und das Methylenchlorid im Vakuum entfernt. Der Rückstand wird bei 95 °C, im Ölpumpenvakuum sublimiert.
Ausbeute: 3,83 g = 43 % der Theorie.

### Beispiel 3 b

### Herstellung von 2,3-Dihydroxy-5-(perfluorhexyl)bicyclo[2.2.1]-heptan

82,4 g (2oo mmol) des Diels-Alder-Umsetzungsproduktes von 3,3,4,4,5,5,6,6,7,7,8,8,8-Tridecafluor-1-octen (Perfluor-hexylethen) und Cyclopentadien werden mit 2o ml Wasser, 1o ml 3o prozentiger Schwefelsäure und 2 g [CH₃N(C₈H₁₇)₃][PO₄{W(O)(O₂)₂}₄] gemischt und 2 ml der insgesamt 34,4 ml (2oo mmol) 35 prozentiges Wasserstoffperoxid zugegeben und unter Rühren auf 6o °C erwärmt. Bei dieser Temperatur wird das restliche Wasserstoffperoxid innerhalb von 3o Minuten zugetropft. Im Verlauf der Reaktion wird die Suspension immer viskoser. Nach 17 Stunden trennt man die organische Phase als untere Phase des Reaktionsgemisches ab, extrahiert die wässerige Phase mit 2oo ml Methylenchlorid und trocknet die vereinigten organischen Phasen mit Natriumsulfat. Beim Abdestillieren des Lösungsmittels scheidet sich ein weißer Feststoff ab, der bei 0 °C mit Heptan gewaschen wird.
Ausbeute: 83,9 g = 94 % der Theorie.

### Beispiel 4

### Herstellung von 2,3-Dihydroxy-5-(perfluoroctyl)bicyclo[2.2.1]heptan

10,2 g (20 mmol) des Diels-Alder-Umsetzungsproduktes von 3,3,4,4,5,5,6,6,7,7,8,8,9,9,10,10,10-Heptadecafluor-1-decen (Perfluoroctylethen) und Cyclopentadien werden mit 20 ml Ameisensäure gemischt und bei einer Temperatur von 50°C 2,6 ml 35 prozentiges Wasserstoffperoxid (30 mmol) zugegeben und 36 Stunden lang bei 50°C gehalten. Das Lösungsmittel wird im Vakuum abgezogen und der Rückstand mit 1oo ml 4 %iger Natronlauge auf 5o °C erwärmt. Hierbei bilden sich zwei Phasen aus. Nach Neutralisation mit Salzsäure wird die wäßrige Phase in 3 Portionen mit insgesamt 2oo ml Methylenchlorid extrahiert. Die Extrakte und die organische Phase werden vereinigt und mit Natriumsulfat getrocknet. Danach wird das Methylenchlorid im Vakuum entfernt. Der Rückstand wird bei 112 °C im Ölpumpenvakuum sublimiert.
Ausbeute: 4,54 g = 43 % der Theorie.

### Beispiel 5

### Herstellung von 2,3-Hydroxy-5-(2,2,3,3,4,4,5,5,6,6,7,7,7-tridecafluorheptyl)bicyclo[2.2.1]heptan

4,3 g (10 mmol) des Diels-Alder-Umsetzungsproduktes von 4,4,5,5,6,6,7,7,8,8,9,9,9-Tridecafluor-1-nonen (Perfluorhexylpropen-1) und Cyclopentadien werden mit 10 ml Wasser, 1 ml 30 prozentiger Schwefelsäure und 200 mg [CH₃N(C₈H₁₇)₃][PO₄{W(O)(O₂)₂}₄] gemischt, 1,7 ml (20 mmol) 35 prozentiges Wasserstoffperoxid zugegeben und unter Rühren auf 60°C erwärmt. Im Verlauf der Reaktion wird die Emulsion immer viskoser. Nach 17 Stunden wird abgekühlt und der ausgefallene weiße Feststoff in Methylenchlorid aufgenommen. Die erhaltene Lösung wird zweimal mit Wasser gewaschen und mit Natriumsulfat getrocknet. Das Lösungsmittel wird im Vakuum entfernt und der Rückstand bei 100°C und 0,1 mbar sublimiert.
Ausbeute: 2,6 g = 55 % der Theorie.

## Patentansprüche (Patentansprüche für folgende(n) Vertragsstaat(en): AT, BE, CH, DE, FR, GB, IT, LI, NL)

1. Fluorsubstituierte vicinale Glykole der allgemeinen Formel 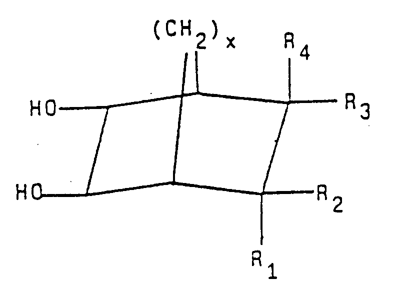
in der R₁ CF₃, F oder H,
R₂ CF₃, C₆F₁₃, C₈F₁₇, CH₂-C₆F₁₃, (CH₂)₂-C₆F₁₈
R₃ F oder H
R₄ F oder H
bedeuten
und x für 0 oder 1 steht.

2. Das fluorsubstituierte vicinale Glykol nach Anspruch 1,
dadurch gekennzeichnet, daß es
2,3-Dihydroxy-5,5-bis(trifluormethyl)bicyclo[2.2.1]heptan. ist.

3. Das fluorsubstituierte vicinale Glykol nach Anspruch 1, dadurch gekennzeichnet, daß es 2,3-Dihydroxy-5,6,6-trifluor-5-(trifluormethyl)bicyclo[2.2.1]heptan ist.

4. Das fluorsubstituierte vicinale Glykol nach Anspruch 1,
dadurch gekennzeichnet, daß es
2,3-Dihydroxy-5-(perfluorhexyl)bicyclo[2.2.1.]heptan ist.

5. Das fluorsubstituierte vicinale Glykol nach Anspruch 1,
dadurch gekennzeichnet, daß es
2,3-Dihydroxy-5-(perfluoroctyl)bicyclo[2.2.1]heptan ist.

6. Das fluorsubstituierte vicinale Glykol nach Anspruch 1,
dadurch gekennzeichnet, daß es
1,2-Dihydroxy-4-(perfluorhexyl)cyclohexan ist.

7. Das fluorsubstituierte vicinale Glykol nach Anspruch 1,
dadurch gekennzeichnet, daß es
2,3-Dihydroxy-5-(2,2,3,3,4,4,5,5,6,6,7,7,7-tridecafluorheptyl)bicyclo[2.2.1]heptan ist.

8. Das fluorsubstituierte vicinale Glykol nach Anspruch 1,
dadurch gekennzeichnet, daß es
2,3-Dihydroxy-5-(3,3,4,4,5,5,6,6,7,7,8,8,8-tridecafluoroctyl)bicyclo[2.2.1]heptan ist.

9. Verfahren zur Herstellung fluorsubstituierter vicinaler Glykole, nach einem der Patentansprüche 1 bis 8, aus dem Umsetzungsprodukt von Butadien oder Cyclopentadien mit fluorsubstituiertem Alken nach der Arbeitsweise einer Diels-Alder-Reaktion, dadurch gekennzeichnet, daß man das Umsetzungsprodukt in Gegenwart einer Säure mit Wasserstoffperoxid bei Temperaturen zwischen 4o und 1oo °C zur Reaktion bringt und das fluorsubstituierte vicinale Glykol isoliert.

10. Verfahren nach Anspruch 9, dadurch gekennzeichnet, daß man 3o bis 7o %iges Wasserstoffperoxid in einer Menge von 1 bis 5 Äquivalenten Oxidationsmittel, bezogen auf das eingesetzte Diels-Alder-Umsetzungsprodukt, einsetzt.

11. Verfahren nach Anspruch 9 oder 10, dadurch gekennzeichnet, daß man als Säure Ameisen-, Essig-, Propionsäure oder wäßrige Schwefelsäure zusammen mit einer katalytischen Menge von [CH₃N(C₈H₁₇)₃][PO₄{W(O)(O₂)₂} ₄] einsetzt.

12. Verfahren nach einem der Ansprüche 9 bis 11, dadurch gekennzeichnet, daß man das Diels-Alder-Umsetzungsprodukt mit der 0,1- bis 1o-fachen Säuremenge vermischt.

13. Verfahren nach einem der Ansprüche 9 bis 12, dadurch gekennzeichnet, daß man die Reaktion in 1 bis 50 gewichtsprozentiger Schwefelsäure durchführt.

14. Verfahren nach einem der Ansprüche 9 bis 13, dadurch gekennzeichnet, daß man 0,1 bis 10 Molprozente [CH₃N(C₈H₁₇)₃]/PO₄{W(O)(O₂)₂}₄], bezogen auf das Diels-Alder Umsetzungsprodukt, einsetzt.

15. Verfahren nach einem der Ansprüche 9 bis 14, dadurch gekennzeichnet, daß man das fluorsubstituierte vicinale Glykol durch eine Sublimation reinigt.

16. Verfahren nach einem der Ansprüche 9 bis 11, dadurch gekennzeichnet, daß man das säurehaltige fluorsubstituierte vicinale Glykol mit Natronlauge versetzt, die wäßrige Phase mit Methylenchlorid extrahiert, den Extrakt mit der organischen Phase vereinigt, trocknet und danach das Methylenchlorid abdestilliert.

17. Verwendung fluorsubstituierter vicinaler Glykole nach einem der Ansprüche 1 bis 16 zur Herstellung von Polyestern, dadurch gekennzeichnet, daß man fluorsubstituierte vicinale Glykole mit Dicarbonsäuren kondensiert.

18. Verwendung fluorsubstituierter vicinaler Glykole nach einem der Ansprüche 1 bis 16 zur Herstellung von Polyestern, dadurch gekennzeichnet, daß man 5 bis 50 Molprozente fluorsubstituierte vicinale Glykole in Mischung mit Glykolen zusammen mit Dicarbonsäuren kondensiert.

## Patentansprüche (Patentansprüche für folgende(n) Vertragsstaat(en): ES)

1. Verfahren zur Herstellung fluorsubstituierter vicinaler Glykole der allgemeinen Formel 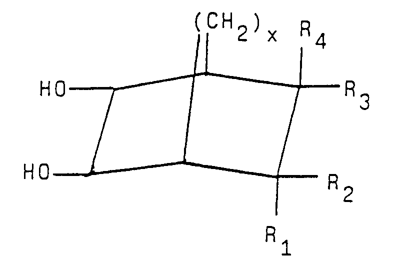 in der R₁ CF₃, F oder H, R₂ CF₃, C₆F₁₃, C₈F₁₇, CH₂-C₆F₁₃, (CH₂)₂C₆F₁₈, R₃ F oder H, R₄ F oder H bedeuten und x für 0 oder 1 steht, aus dem Umsetzungsprodukt von Butadien oder Cyclopentadien mit fluorsubstituiertem Alken nach der Arbeitsweise einer Diels-Alder-Reaktion, dadurch gekernzeichnet, daß man das Umsetzungsprodukt in Gegenwart einer Säure mit Wasserstoffperoxid bei Temperaturen zwischen 40 und 100 °C zur Reaktion bringt und das fluorsubstituierte vicinale Glykol isoliert.

2. Verfahren nach Anspruch 1, dadurch gekennzeichnet, daß man 30 bis 70 %iges Wasserstoffperoxid in einer Menge von 1 bis 5 Äquivalenten Oxidationsmittel, bezogen auf das eingesetzte Diels-Alder-Umsetzungsprodukt, einsetzt.

3. Verfahren nach Anspruch 1 oder 2, dadurch gekennzeichnet, daß man als Säure Ameisen-, Essig-, Propionsäure oder wäßrige Schwefelsäure zusammen mit einer katalytischen Menge von [CH₃N(C₈H₁₇)₃]PO₄{W(O)(O₂)₂}₄] einsetzt.

4. Verfahren nach einem der Ansprüche 1 bis 3, dadurch gekennzeichnet, daß man das Diels-Alder-Umsetzungsprodukt mit der 0,1- bis 10-fachen Säuremenge vermischt.

5. Verfahren nach einem der Ansprüche 1 bis 4, dadurch gekennzeichnet, daß man die Reaktion in 1 bis 50 gewichtsprozentiger Schwefelsäure durchführt.

6. Verfahren nach einem der Ansprüche 1 bis 5, dadurch gekennzeichnet, daß man 0,1 bis 10 Molprozente [CH₃N(C₈H₁₇)₃][PO₄{W(O)(O₂)₂}₄], bezogen auf das Diels-Alder-Umsetzungsprodukt, einsetzt.

7. Verfahren nach einem der Ansprüche 1 bis 6, dadurch gekennzeichnet, daß man das fluorsubstituierte vicinale Glykol durch eine Sublimation reinigt.

8. Verfahren nach einem der Ansprüche 1 bis 7, dadurch gekennzeichnet, daß man das säurehaltige fluorsubstituierte vicinale Glykol mit Natronlauge versetzt, die wäßrige Phase mit Methylenchlorid extrahiert, den Extrakt mit der organischen Phase vereinigt, trocknet und danach das Methylenchlorid abdestilliert.

9. Verwendung fluorsubstituierter vicinaler Glykole, hergestellt nach einem der Ansprüche 1 bis 8 zur Herstellung von Polyestern, dadurch gekennzeichnet, daß man fluorsubstituierte vicinale Glykole mit Dicarbonsäuren kondensiert.

10. Verwendung fluorsubstituierter vicinaler Glykole, hergestellt nach einem der Ansprüche 1 bis 8 zur Herstellung von Polyestern, dadurch gekennzeichnet, daß man 5 bis 50 Molprozente fluorsubstituierte vicinale Glykole in Mischung mit Glykolen zusammen mit Dicarbonsäuren kondensiert.

## Claims (Claims for the following Contracting State(s): AT, BE, CH, DE, FR, GB, IT, LI, NL)

1. A fluorine-substituted vicinal glycol of the formula 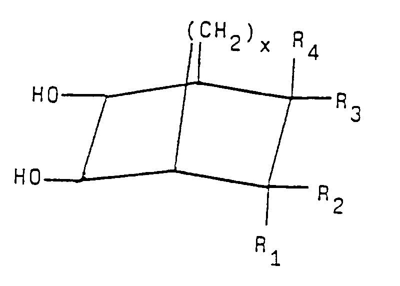
in which R₁ is CF₃, F or H,
R₂ is CF₃, C₆F₁₃, C₈F₁₇, CH₂-C₆F₁₃, (CH₂)₂-C₆F₁₈
R₃ is F or H
R₄ F or H
and x is 0 or 1.

2. A fluorine-substituted vicinal glycol as claimed in claim 1, being 2,3-dihydroxy-5,5-bis(trifluoromethyl)bicyclo[2.2.1]heptane.

3. A fluorine-substituted vicinal glycol as claimed in claim 1, being 2,3-dihydroxy-5,6,6-trifluoro-5-(trifluoromethyl)bicyclo[2.2.1]heptane.

4. A fluorine-substituted vicinal glycol as claimed in claim 1, being 2,3-dihydroxy-5-(perfluorohexyl)bicyclo[2.2.1]heptane.

5. A fluorine-substituted vicinal glycol as claimed in claim 1, being 2,3-dihydroxy-5-(perfluorooctyl)bicyclo[2.2.1]heptane.

6. A fluorine-substituted vicinal glycol as claimed in claim 1, being 1,2-dihydroxy-4-(perfluorohexyl)cyclohexane.

7. A fluorine-substituted vicinal glycol as claimed in claim 1, being 2,3-dihydroxy-5-(2,2,3,3,4,4,5,5,6,6,7,7,7-tridecafluoroheptyl)bicyclo[2.2.1]heptane.

8. A fluorine-substituted vicinal glycol as claimed in claim 1, being 2,3-dihydroxy-5-(3,3,4,4,5,5,6,6,7,7,8,8,8-tridecafluorooctyl)bicyclo[2.2.1]heptane.

9. A method for the preparation of a fluorine-substituted vicinal glycol as claimed in any one of claims 1 to 8, from the reaction product of butadiene or cyclopentadiene with a fluorine-substituted alkene according to the procedure of a Diels-Alder reaction, which comprises reacting the reaction product with hydrogen peroxide in the presence of an acid at temperatures between 40 and 100°C and isolating the fluorine-substituted vicinal glycol.

10. The method as claimed in claim 9, wherein hydrogen peroxide of 30 to 70% strength is used in an amount of 1 equivalent to 5 equivalents of oxidant, based on the Diels-Alder reaction product used.

11. The method as claimed in claim 9 or 10, wherein the acid used is formic acid, acetic acid, propionic acid or agueous sulfuric acid, together with a catalytic amount of [CH₃N(C₈H₁₇)₃][PO₄{W(O)(O₂)₂}₄].

12. The method as claimed in any one of claims 9 to 11, wherein the Diels-Alder reaction product is mixed with a 0.1- to 10-fold amount of acid.

13. The method as claimed in any one of claims 9 to 12, wherein the reaction is carried out in sulfuric acid of 1 to 50 percent by weight strength.

14. The method as claimed in any one of claims 9 to 13, wherein 0.1 to 10 mol percent of [CH₃N(C₈H₁₇)₃][PO₄{W(O)(O₂)₂}₄] are used, based on the Diels-Alder reaction product.

15. The method as claimed in any one of claims 9 to 14, wherein the fluorine-substituted vicinal glycol is purified by a sublimation.

16. The method as claimed in any one of claims 9 to 11, wherein the acid-containing fluorine-substituted vicinal glycol is treated with aqueous sodium hydroxide, the aqueous phase is extracted with methylene chloride, the extract is combined with the organic phase and dried, and the methylene chloride is then distilled off.

17. Use of a fluorine-substituted vicinal glycol as claimed in any one of claims 1 to 16 for the preparation of polyesters, wherein fluorine-substituted vicinal glycols are condensed with dicarboxylic acids.

18. Use of a fluorine-substituted vicinal glycol as claimed in any one of claims 1 to 16 for the preparation of polyesters, wherein 5 to 50 mol percent of fluorine-substituted vicinal glycols mixed together with glycols are condensed with dicarboxylic acids.

## Claims (Claims for the following Contracting State(s): ES)

1. A method for the preparation of a fluorine-substituted vicinal glycol of the formula 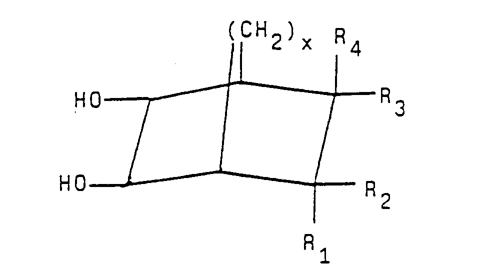 in which R₁ is CF₃, F or H, R₂ is CF₃, C₆F₁₃, C₈F₁₇, CH₂-C₆F₁₃, (CH₂)₂-C₆F₁₈, R₃ is F or H, R₄ is F or H and x is 0 or 1, from the reaction product of butadiene or cyclopentadiene with a fluorine-substituted alkene according to the procedure of a Diels-Alder reaction, which comprises reacting the reaction product with hydrogen peroxide in the presence of an acid at temperatures between 40 and 100°C and isolating the fluorine-substituted vicinal glycol.

2. The method as claimed in claim 1, wherein hydrogen peroxide of 30 to 70% strength is used in an amount of 1 equivalent to 5 equivalents of oxidant, based on the Diels-Alder reaction product used.

3. The method as claimed in claim 1 or 2, wherein the acid used is formic acid, acetic acid, propionic acid or aqueous sulfuric acid, together with a catalytic amount of [CH₃N(c₈H₁₇)₃][PO₄{W(O)(O₂)₂}₄].

4. The method as claimed in any one of claims 1 to 3, wherein the Diels-Alder reaction product is mixed with a 0.1- to 10-fold amount of acid.

5. The method as claimed in any one of claims 1 to 4, wherein the reaction is carried out in sulfuric acid of 1 to 50 percent by weight strength.

6. The method as claimed in any one of claims 1 to 5, wherein 0.1 to 10 mol percent of [CH₃N(C₈H₁₇)₃][PO₄{W(O)(O₂)₂}₄] are used, based on the Diels-Alder reaction product.

7. The method as claimed in any one of claims 1 to 6, wherein the fluorine-substituted vicinal glycol is purified by a sublimation.

8. The method as claimed in any one of claims 1 to 7, wherein the acid-containing fluorine-substituted vicinal glycol is treated with aqueous sodium hydroxide, the aqueous phase is extracted with methylene chloride, the extract is combined with the organic phase and dried, and the methylene chloride is then distilled off.

9. The use of a fluorine-substituted vicinal glycol prepared as claimed in any one of claims 1 to 8 for the preparation of polyesters, wherein fluorine-substituted vicinal glycols are condensed with dicarboxylic acids.

10. The use of a fluorine-substituted vicinal glycol prepared as claimed in any one of claims 1 to 8 for the preparation of polyesters, wherein 5 to 50 mol percent of fluorine-substituted vicinal glycols mixed together with glycols are condensed with dicarboxylic acids.

## Revendications (Revendications pour l'(les) Etat(s) contractant(s) suivant(s): AT, BE, CH, DE, FR, GB, IT, LI, NL)

1. Glycols vicinaux fluorés de formule générale 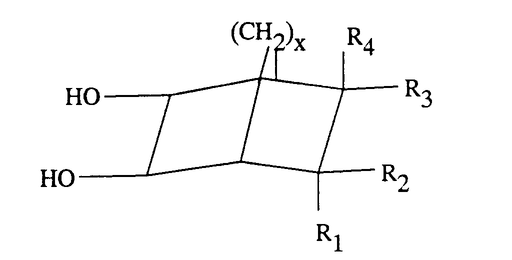
dans laquelle R₁ représente CF₃, F ou H,
R₂ représente CF₃, C₆F₁₃, C₈F₁₇, CH₂-C₆F₁₃, (CH₂)₂-C₆F₁₈,
R₃ représente F ou H,
R₄ représente F ou H, et
x est égal à 0 ou 1,

2. Un glycol vicinal fluoré selon la revendication 1, caractérisé en ce qu'il consiste en le 2,3-dihydroxy-5,5-bis(trifluorométhyl)bicyclo[2.2.1]heptane.

3. Un glycol vicinal fluoré selon la revendication 1, caractérisé en ce qu'il consiste en le 2,3-dihydroxy-5,6,6-trifluoro-5-(trifluorométhyl)bicyclo[2.2.1]heptane.

4. Un glycol vicinal fluoré selon la revendication 1, caractérisé en ce qu'il consiste en le 2,3-dihydroxy-5-[perfluorhexyl)bicyclo[2.2.1]heptane.

5. Un glycol vicinal fluoré selon la revendication 1, caractérisé en ce qu'il consiste en le 2,3-dihydroxy-5-(perfluoroctyl)bicyclo[2.2.1]heptane.

6. Un glycol vicinal fluoré selon revendication 1, caractérisé en ce qu'il consiste en le 1,2-dihydroxy-4-perfluorhexyl)cyclohexane.

7. Un glycol vicinal fluoré selon la revendication 1, caractérisé en ce qu'il consiste en le 2,3-dihydroxy-5-(2,2,3,3,4,4,5,5,6,6,7,7,7-tridécafluorheptyl)bicyclo[2.2.1]heptane.

8. Un glycol vicinal fluoré selon revendication 1, caractérisé en ce qu'il consiste en le 2,3-dihydroxy-5-(3,3,4,4,5,5,6,6,7,7,8,8,8-tridécafluoroctyl)bicyclo[2.2.1]heptane.

9. Procédé de préparation de glycols vicinaux fluorés selon une des revendications 1 à 8, à partir du produit de la réaction de Diels-Alder entre la butadiène ou le cyclopentadiène et un alcène fluoré, caractérisé en ce que l'on fait réagir ce produit en présence d'un acide avec du peroxyde d'hydrogène à des températures de 40 à 100°C et on isole le glycol vicinal fluoré.

10. Procédé selon revendication 9, caractérisé en ce que l'on utilise du peroxyde d'hydrogène à une concentration de 30 à 70 % en quantité de 1 à 5 équivalents de l'agent oxydant par rapport au produit de la réaction de Diels-Alder mis en oeuvre.

11. Procédé selon revendication 9 ou 10, caractérisé en ce que l'acide utilisé est l'acide formique, l'acide acétique, l'acide propionique ou l'acide sulfurique aqueux, avec une quantité catalytique de [CH₃N(C₈H₁₇)₃][PO₄{W(O)(O₂)₂}₄].

12. Procédé selon l'une des revendications 9 à 11, caractérisé en ce que l'on mélange le produit de la réaction de Diels-Alder avec 0,1 à 10 fois son poids d'acide.

13. Procédé selon une des revendications 9 à 12, caractérisé en ce que l'on effectue la réaction dans l'acide sulfurique à une concentration de 1 à 50 % en poids.

14. Procédé selon une des revendications 9 à 13, caractérisé en ce que l'on utilise de 0,1 à 10 mol % de [CH₃N(C₈H₁₇)₃][PO₄{W(O)(O₂)₂}₄] par rapport au produit de la réaction de Diels-Alder.

15. Procédé selon l'une des revendications 9 à 14, caractérisé en ce que l'on purifie le glycol vicinal fluoré par une sublimation.

16. Procédé selon une des revendications 9 à 11, caractérisé en ce que l'on ajoute de la lessive de soude au glycol vicinal fluoré qui contient de l'acide, on extrait la phase aqueuse par le chlorure de méthylène, on combine l'extrait avec la phase organique, on sèche puis on distille le chlorure de méthylène.

17. Utilisation des glycols vicinaux fluorés selon une des revendications 1 à 16, pour la préparation de polyesters, caractérisée en ce que l'on condense les glycols vicinaux fluorés avec des acides dicarboxyliques.

18. Utilisation des glycols vicinaux fluorés selon l'une des revendications 1 à 16, pour la préparation de polyesters, caractérisée en ce que l'on condense avec des acides dicarboxyliques de 5 à 50 mol % de glycols vicinaux fluorés en mélange avec des glycols.

## Revendications (Revendications pour l'(les) Etat(s) contractant(s) suivant(s): ES)

1. Procédé de préparation de glycols vicinaux fluorés répondant à la formule générale 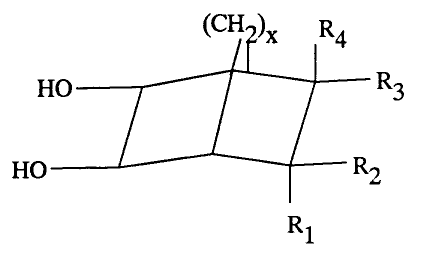 dans laquelle R₁ représente CF₃, F ou H, R₂ représente CF₃, C₆F₁₃, C₈F₁₇, CH₂-C₆F₁₃, (CH₂)₂-C₆F₁₈, R₃ représente F ou H, R₄ représente F ou H et x est égal à 0 ou 1, à partir du produit de la réaction de Diels-Alder entre le butadiène ou le cyclopentadiène et un alcène fluoré, caractérisé en ce que l'on fait réagir ce produit en présence d'un acide avec le peroxyde d'hydrogène à des températures de 40 à 100°C et on isole le glycol vicinal fluoré.

2. Procédé selon la revendication 1, caractérisé en ce que l'on utilise le peroxyde d'hydrogène à une concentration de 30 à 70 % en quantité de 1 à 5 équivalents d'agent oxydant par rapport au produit de la réaction de Diels-Alder mis en oeuvre.

3. Procédé selon la revendication 1 ou 2, caractérisé en ce que l'acide utilisé est l'acide formique, l'acide acétique, l'acide propionique ou l'acide sulfurique aqueux, avec une quantité catalytique de [CH₃N(C₈H₁₇)₃][PO₄{W(O)(O₂)₂}₄].

4. Procédé selon une des revendications 1 à 3, caractérisé en ce que l'on mélange le produit de la réaction de Diels-Alder avec 0,1 à 10 fois son poids d'acide.

5. Procédé selon une des revendications 1 à 4, caractérisé en ce que l'on effectue la réaction dans l'acide sulfurique à une concentration de 1 à 50 % en poids.

6. Procédé selon une des revendications 1 à 5, caractérisé en ce que l'on utilise de 0,1 à 10 mol % de [CH₃N(C₈H₁₇)₃][PO₄{W(O)(O₂)₂}₄] par rapport au produit de la réaction de Diels-Alder.

7. Procédé selon une des revendications 1 à 6, caractérisé en ce que l'on purifie le glycol vicinal fluoré par une sublimation.

8. Procédé selon une des revendications 1 à 7, caractérisé en ce que l'on ajoute de la lessive de soude au glycol vicinal fluoré qui contient de l'acide, on extrait la phase aqueuse par le chlorure de méthylène, on combine l'extrait avec la phase organique, on sèche puis on distille le chlorure de méthylène.

9. Utilisation de glycols vicinaux fluorés préparés selon l'une des revendications 1 à 8 pour la préparation de polyesters, caractérisée en ce que l'on condense les glycols vicinaux fluorés avec des acides dicarboxyliques.

10. Utilisation des glycols vicinaux fluorés préparés selon une des revendications 1 à 8 pour la préparation de polyesters, caractérisée en ce que l'on condense avec des acides dicarboxyliques de 5 à 50 mol % des glycols vicinaux fluorés en mélange avec des glycols.
